# EUROPEAN PATENT SPECIFICATION

(11) **EP 0 345 628 B1**
(45) Date of publication and mention of the grant of the patent: **12.05.1993**
(21) Application number: 89109891.5
(22) Date of filing: 01.06.1989
(51) Int. Cl.: A61K 9/26, A61K 9/20, A61K 31/55

(54) **Solid pharmaceutical dosage in tablet triturate form and method of producing same**
Feste Arzneidosis in Form eines Tablettentriturats sowie Verfahren zu deren Herstellung
Dose pharmaceutique solide sous forme d'une trituration de comprimé et son procédé de préparation

(30) Priority: 07.06.1988 US 203396; 18.05.1989 US 352799
(43) Date of publication of application: 13.12.1989
(73) Proprietor: ABBOTT LABORATORIES, Abbott Park, Illinois 60064-3500 (US)
(72) Inventor: Van Scoik, Kurt Gard, Grayslake Illinois 60030 (US)
(74) Representative: Modiano, Guido, Dr.-Ing.

(56) References cited:
- EP-A- 0 139 460
- EP-A- 0 177 368
- EP-A- 0 251 680
- EP-A- 0 273 890
- US-A- 4 013 784

## Description

### Technical Field

This invention relates to a specific tablet triturate suitable for oral administration of a pharmaceutically active ingredient and in particular to a tablet triturate that dissolves relatively quickly in the buccal cavity and masks the taste of the active ingredient as well.

### Background of the Invention

Patient compliance with a prescribed regimen of taking orally administered drugs is necessary for effective treatment. However, this compliance is often adversely affected by drugs which are not palatable. Reduced compliance can also occur with a pediatric or geriatric patient who will not or cannot swallow solid tablets. Similar difficulty can occur in veterinary treatment in that animals can be uncooperative about taking the drug in tablet form.

Conventional forms of drugs for oral administration include direct compression tablets, sublingual tablets, spray congealed powders and triturate tablets. These forms, however, do not provide the advantage of the present specific tablet triturate of this invention.

Tablets which are formed by direct compression are ill suited for the rapid administration of drugs in that compressed tablets do not disintegrate or dissolve fast enough. Furthermore these tablets are difficult for certain patients to swallow.

Sublingual tablets are designed for rapid administration of medication and are placed beneath the tongue and held there until absorption of the drug has taken place through the mucous membranes. These tablets do not improve patient compliance in the above described problem areas, however. Inasmuch as the tablet is retained beneath the tongue to release the drug for absorption in the buccal cavity, an unpalatable sensation is experienced by the patient. In the case of an infant, or psychiatric patient, the patient may also spit the tablet out.

The process of spray congealing involves cooling (or congealing) of melted substances in the form of fine particles during their travel from a spray nozzle to a distant vicinity of a spray chamber held at a temperature below the melting temperatures of the substances. If a slurry of material insoluble in the melted mass of a congealing substance is spray congealed, the insoluble material is coated with the congealing substance. This method provides taste masking. However, this process does not provide tablets for ease of oral administration of the drug. Furthermore, the congealing substance is usually a fatty acid or monoglycerides, diglycerides, or triglycerides of edible fatty acids.

Trituration is the mixing of powders using a grinding action, such as by a mortar and pestle, followed by moistening of the powders. This moistened powder is then molded into tablet form and dried. Tablets thus produced do not exhibit taste masking characteristics.

The present invention provides a solid pharmaceutical dosage in tablet triturate form which avoids the shortcomings of the prior art and is both readily dissolvable and masks the taste of the active ingredient.

### Summary of the Invention

The present invention contemplates a solid pharmaceutical dosage in tablet triturate form which dissolves quickly and masks the taste of the active ingredient.

This tablet triturate form includes a porous cementatory network having discrete particles encasing an active ingredient dispersed throughout the network. The network is substantially constituted by a water-soluble but ethanol-insoluble carbohydrate. The discrete particles include a solid, water-soluble but triglyceride-insoluble active ingredient provided with a triglyceride coating.

These discrete particles are produced by first suspending the active ingredient in a melted triglyceride vehicle. The triglyceride vehicle may also contain polymers, such as polyethylene glycol (PEG); sodium bicarbonate; and emulsifiers, such as lecithin, to modify the rate and extent of drug release from the particles. This suspension is then spray congealed to form solid discrete particles having the active ingredient, polymer, sodium bicarbonate, and emulsifier all of which are encapsulated in the triglyceride vehicle.

The tablet triturate contemplated by the present invention is produced by admixing the carbohydrate, the discrete particles and a temporary liquid binder such as a water-ethanol admixture in an amount sufficient to form a damp mass. The resulting damp mass is shaped into a tablet and subsequently dried to produce the desired tablet triturate.

The present dosage form is particularly well suited for the administration of bitter-tasting medication such as estazolam and clorazepate dipotassium.

Numerous other advantages and features of the present invention will become readily apparent from the following detailed description of the invention, the accompanying examples, and the appended claims.

### Detailed Description of the Preferred Embodiments

While this invention is susceptible to embodiment in many different forms, preferred embodiments of the invention are shown.

The present invention is directed to a solid pharmaceutical dosage in tablet triturate form. The relatively quick dissolution, acceptable taste and acceptable stability of the tablet triturate as a dosage form makes it suitable for oral administration of pharmaceutically active ingredients that have an unpleasant taste.

This tablet triturate dosage form has a porous cementatory network substantially constituted by a water-soluble but ethanol-insoluble carbohydrate. Distributed substantially uniformly throughout the network are discrete particles comprising the solid, water-soluble but triglyceride-insoluble active ingredient and, where applicable, the polymer, sodium bicarbonate, and emulsifier. These particles have a triglyceride coating which is insoluble in water, ethanol, saliva or combinations thereof. As the particles are not significantly dissolved in the buccal cavity, taste masking of the active ingredient is realized. Absorption of the discrete particles occurs when they reach the digestive tract where the active ingredient is released for systemic delivery. The triglyceride coating constitutes a major portion of the weight of each discrete particle, usually 55 to 80 percent by weight of each discrete particle.

Suitable carbohydrates for the present purposes are monosaccharides such as fructose and dextrose, disaccharides such as lactose and sucrose, as well as combinations thereof. Preferably the carbohydrate is in powder form and having a particle size distribution of less than 100 µm in diameter. A preferred carbohydrate is lactose, more preferably lactose monohydrate.

Suitable polymers for the present purposes include PEG 200, 300, 400, 3350, and 8000, where the numerical value approximates the molecular weight. In the context of the present invention, the lower molecular weight polymers are preferred as they will produce a faster dissolution rate.

In addition to the aforementioned monosaccharides and disaccharides, a carbohydrate such as a sugar alcohol, e .g., mannitol and sorbitol, and admixtures thereof can be present in the cementatory network in an amount up to 20 percent by weight of the total amount of carbohydrates present.

The above identified carbohydrates form a porous cementatory network which is fast-dissolving in the buccal cavity and which serves to retain therewithin the spray congealed active ingredient or ingredients.

The active ingredients suitable for preparation of the present dosage forms are not temperature sensitive at the melting temperature of the triglyceride employed. Suitable active ingredients are those which are stable in melted and room temperature triglyceride. Illustrative such active ingredients are estazolam (8-chloro-6-phenyl-4H-s-triazolo [4,3-a][1.4] benzodiazepine), clorazepate dipotassium (7-chloro-2, 3-dihydro-2 and 2-dihydroxy-5-phenyl-1H-1,4-benzodiazepine-3-carboxylic acid dipotassium).

The triglyceride is a tri-ester of a fatty acid and glycerol represented by the general formula CH₂(OOCR₁)CH(OOCR₂)CH₂(OOCR₃), wherein R₁, R₂, and R₃ are independently selected from fatty acid residues, usually of different chain lengths. These fatty acid residues are at least C₁₆, preferably at least C₁₈ in carbon chain length. Triglycerides of this size possess a melting temperature which is satisfactory for the present purposes. Generally, illustrative triglycerides are hydrogenated vegetable oil such as hydrogenated cottonseed oil and hydrogenated animal oils. Specifically, illustrative triglycerides include tristearin and palmitodistearin. These triglycerides have good storage stability but are eventually broken down in the small intestine, thereby releasing any remaining active ingredient for systemic absorption.

Other optional ingredients that can be incorporated into the present tablet triturate dosage forms include known pharmaceutically acceptable excipients, such as flavoring, sweetening agents, and coloring agents, for example, peppermint flavor or aspartame.

The discrete particles containing the active ingredient are formed by a spray congealing process. First, a triglyceride having a melting temperature below the temperature at which the active ingredient melts is selected and converted to liquid phase by heating. If sodium bicarbonate is utilized, the sodium bicarbonate can be added to the liquid phase at this point. The active ingredient, in powder form, is then admixed with the triglyceride melt and homogeneously dispersed therein. If polymer and emulsifier are utilized, the polymer and the emulsifier can be mixed together and circulated through the system at this point. The resulting mixture is a suspension of the active ingredient, the polymer, the emulsifier, and the sodium bicarbonate in the triglyceride melt. Next, the admixture is spray congealed in a conventional manner to produce discrete particles of suitable particle size in which the active ingredient, the Polymer, sodium bicarbonate, and the emulsifier are encased in a triglyceride envelope.

To produce the tablet triturate dosage form, the solid components in desired proportions are combined in a suitable vessel. A small amount of a volatizable, temporary, liquid binder having limited solubility for the carbohydrate, e.g., a water-ethanol admixture, sufficient to form a slightly damp mass of the solid components is added. This mass is then mixed to substantial homogeneity. The produced homogeneous mass is forced into a mold having tablet-shaped holes in a plate. The tablets are solidified by evaporating the volatizable liquid binder present, thereby yielding the porous tablet triturate dosage forms of the present invention. These tablet triturates have a rigid self-supporting structure.

When this tablet triturate is inserted in the buccal cavity, the carbohydrate cementatory network of the present tablet triturate is readily and rapidly dissolved in approximately 5 seconds by the saliva present in the buccal cavity. However, the discrete particles containing the active ingredient are substantially unaffected by the saliva but can be readily swallowed without the taste of the encapsulated active ingredients becoming manifest to the patient.

In the dosage form of the present invention, the weight ratio of carbohydrate:discrete particles preferably is in the range of 4:1 to 100:1. The discrete particles can constitute 1 percent to 25 percent by weight of the dosage form.

The weight ratio of active ingredient triglyceride is in the range of 1:1 to 1:5, preferably 1:2 to 1:4.

The particle size of the active ingredient usually is in the range of 75 µm to 150 µm (preferably 100 µm to 150 µm in diameter).

The maximum particle size of the discrete particles is such that the discrete particles are not discernible by a patient when they are present in the buccal cavity. A sensation of grittiness or sandiness is thereby avoided. Suitable discrete particle sizes are preferably less than 180 µm in diameter.

A ratio of liquid binder to dry solid components which is sufficient to yield a damp homogeneous mass is 10-15:100 (v/m), respectively.

The shape of the tablet triturate is not critical to the performance of the tablet. Standard manufacturing and handling, as well as administration considerations determine the ultimate shape of the tablet.

Illustrative of the dosage form of the invention is a dosage form wherein the carbohydrate is lactose, the active ingredient is estazolam, the triglyceride is hydrogenated cottonseed oil, the polymer is polyethylene glycol of molecular weight 300 and the emulsifier is lecithin, wherein the carbohydrate is 94 percent of the total weight of the tablet.

The following examples further illustrate a dosage form of the present invention as well as a method of making the same.

### EXAMPLE 1

The water-soluble but triglyceride-insoluble active ingredient utilized is crystalline estazolam. The triglyceride utilized is hydrogenated cottonseed oil.

The cottonseed oil, in solid flake form, is placed in a stainless steel vessel and heated to a temperature in the range of 62 to 66° C. Sufficient heat is supplied to melt the oil. The temperature of the molten oil is maintained above the melting temperature of the oil but below the melting temperature of estazolam. Estazolam in powder form is added to the molten oil in the vessel while maintaining vigorous agitation. A commercially available homogenizer is used as an agitator to ensure suspension and distribution of estazolam crystals substantially uniformly throughout the oil. The estazolam particle size is in a range of 75 to 150 µm in diameter.

To ensure satisfactory coating of the estazolam, the weight ratio of estazolam:cottonseed oil in the produced suspension is 1:4.

After the estazolam is adequately distributed into the molten oil, the resulting suspension is pumped from the vessel using a sanitary food-grade pump through heated hoses to a spray gun located inside of a fluid-bed dryer. The spray gun and the air feed to the spray gun are also heated to prevent solidification of the molten suspension during this stage of the manufacturing process. As the molten suspension is forced through the spray gun, a liquid suspension stream is broken up into fine droplets by the heated atomizing air which droplets consist essentially of crystals of estazolam enclosed in the hydrogenated cottonseed oil droplet. These droplets contact cool air drawn into the fluid-bed dryer and solidify as discrete particles. The discrete particles are then collected from the bottom of the fluid-bed dryer as a free flowing powder which is colorless to off-white in color. This material is then classified into desired particle size ranges, e.g., smaller than 80 mesh (177 µm in diameter), using pharmaceutical sieves. Discrete particles having a relatively narrow particle size distribution in the range of 80 to 175 µm are selected for incorporation into the dosage forms.

To manufacture the tablet triturate dosage form, lactose monohydrate is combined with various flavor and/or sweetening agents along with the produced discrete particles. All components are in powder form. An illustrative composition is presented in TABLE I, below.

**TABLE I**

| Composition of a Tablet Triturate Form | |
|---|---|
| Component | Weight, mg |
| Discrete particles* | 10 |
| Lactose monohydrate | 85 |
| Sweetening agent | 3 |
| Flavoring agent | 2 |

| | |
|---|---|
| * The composition of the discrete particles is estazolam:hydrogenated cottonseed oil in a weight ratio of 1:4. | |

The foregoing components are admixed in a suitable vessel. A relatively small amount of a temporary liquid binder, sufficient to dampen the admixture and form a damp mass is added. The liquid binder utilized in this instance is a 60:40 (v/v) admixture of ethanol USP:distilled water. However, the ethanol/water liquid binder may be adjusted to other proportions, or may be comprised of water alone. The amount of liquid binder utilized was 14 milliliters for 100 grams of dry components.

The formed damp mass is next placed in tablet shaped holes in a stainless steel plate to mold tablets. The molded tablets are then removed from the plate and dried.

Machines suitable for manufacturing the tablet triturate dosage form of the present invention are commercially available, inter alia, from Vector-Colton Inc., Marion, Iowa.

### EXAMPLE 2

Cottonseed oil is heated as described in Example 1. Sodium bicarbonate and estazolam are added to the molten oil in the vessel while vigorous agitation is maintained as previously described. PEG 300 and lecithin are mixed together and added to the molten suspension. The resulting suspension is thereafter processed as described in Example 1.

The composition of the discrete particles is illustrated in Table II below. In this case, the composition of the discrete particles is calculated to be 25 percent estazolam.

**TABLE II**

| Composition of Discrete Particles | |
|---|---|
| Component | Weight, mg. |
| estazolam | 25 |
| sodium bicarbonate | 7.5 |
| lecithin | 10 |
| hydrogenated cottonseed oil | 52.5 |
| PEG 300* | 5 |

| | |
|---|---|
| * PEG 300 is available from Union Carbide | |

## Claims (Claims for the following Contracting State(s): BE, CH, DE, FR, GB, IT, LI, NL, SE)

1. A solid pharmaceutical dosage in tablet triturate form, suitable for oral administration, and comprising
a porous cementatory network substantially constituted by a water-soluble but ethanol-insoluble carbohydrate; and
discrete particles comprising a solid, water-soluble but triglyceride-insoluble active ingredient, having a triglyceride coating, substantially uniformly distributed throughout said network, said triglyceride coating being insoluble in water, ethanol, Saliva or combinations thereof;
said triglyceride coating constituting a major portion of the weight of each said discrete particle.

2. The dosage form in accordance with claim 1 wherein the active ingredient is estazolam.

3. The dosage form in accordance with Claim 2 wherein the discrete particles further include an emulsifier and a polymer.

4. The dosage form in accordance with Claim 3 wherein the discrete particles further include sodium bicarbonate.

5. The dosage form in accordance with Claim 4 wherein the discrete particles comprise estazolam as the active ingredient, hydrogenated cottonseed oil as the triglyceride, polyethylene glycol of molecular weight 300 as the polymer, lecithin as the emulsifier and sodium bicarbonate.

6. A solid pharmaceutical dosage in tablet triturate form, suitable for oral administration, and comprising
a porous cementatory network substantially constituted by a water-soluble but ethanol-insoluble carbohydrate; and
discrete particles comprising a solid, water-soluble but triglyceride-insoluble active ingredient, a polymer, sodium bicarbonate, and an emulsifier, said particles having a triglyceride coating, said triglyceride coating being insoluble in water, ethanol, saliva or combinations thereof; and
said triglyceride coating constituting a major portion of the weight of each coated particle, said triglyceride coated particles being substantially uniformly distributed throughout said network.

7. The dosage form in accordance with claim 1 wherein the cementatory network is substantially constituted by lactose and said discrete particles comprise estazolam enveloped in hydrogenated cottonseed oil.

8. The dosage form in accordance with claim 1 wherein the carbohydrate is lactose, the active ingredient is estazolam, the triglyceride is hydrogenated cottonseed oil, the polymer is polyethylene glycol of molecular weight 300, and the emulsifier is lecithin, wherein the carbohydrate is 94 percent of the total weight of the tablet.

9. The dosage form in accordance with Claim 1 wherein the weight ratio of carbohydrate: discrete particles is from 4:1 to 100:1.

10. The dosage form in accordance with Claim 1 wherein the triglyceride coating masks the taste of the active ingredient.

11. A method of manufacturing a solid pharmaceutical dosage in tablet triturate form, suitable for oral administration which comprises:
admixing a particulate water-soluble but ethanol-insoluble carbohydrate with discrete particles containing a solid, water-soluble but triglyceride-insoluble active ingredient enveloped in a triglyceride coating, said triglyceride coating being insoluble in water, ethanol, saliva or combinations thereof and constituting a major portion of the weight of each said discrete particle;
combining the resulting admixture with a volatilizable liquid binder in which said carbohydrate has limited solubility in an amount sufficient to form a moldable mass;
shaping said moldable mass into tablets; and drying said shaped tablets to a rigid structure.

12. A method of manufacturing a solid pharmaceutical dosage in tablet triturate form, suitable for oral administration which comprises:
admixing a particulate water-soluble but ethanol insoluble carbohydrate with discrete particles containing a solid, water-soluble but triglyceride-insoluble active ingredient, a polymer, an emulsifier, and sodium bicarbonate enveloped in a triglyceride coating, said triglyceride coating being insoluble in water, ethanol, saliva or combinations thereof and constituting a major portion of the weight of each said discrete particle; and,
tabletting the produced admixture.

13. A method of manufacturing a tablet triturate dosage form, comprising:
admixing a particulate water-soluble but ethanol-insoluble carbohydrate with discrete particles containing a solid, water-soluble but triglyceride-insoluble active ingredient enveloped in a triglyceride coating.

## Claims (Claims for the following Contracting State(s): ES, GR)

1. A method of manufacturing a solid pharmaceutical dosage in tablet triturate form, suitable for oral administration which comprises:
admixing a particulate water-soluble but ethanol-insoluble carbohydrate with discrete particles comprising a solid, water-soluble but triglyceride-insoluble active ingredient enveloped in a triglyceride coating, said triglyceride coating being insoluble in water, ethanol, saliva or combinations thereof and constituting a major portion of the weight of each said discrete particle;
combining the resulting admixture with a volatilizable liquid binder in which said carbohydrate has limited solubility in an amount sufficient to form a moldable mass;
shaping said moldable mass into tablets; and drying said shaped tablets to a rigid structure.

2. The method in accordance with claim 1 wherein the active ingredient is estazolam.

3. The method in accordance with Claim 2 wherein the discrete particles further include an emulsifier and a polymer.

4. The method in accordance with Claim 3 wherein the discrete particles further include sodium bicarbonate.

5. The method in accordance with Claim 4 wherein the discrete particles comprise estazolam as the active ingredient, hydrogenated cottonseed oil as the triglyceride, polyethylene glycol of molecular weight 300 as the polymer, lecithin as the emulsifier and sodium bicarbonate.

6. The method in accordance with Claim 1 wherein the weight ratio of carbohydrate: discrete particles is from 4:1 to 100:1.

7. The method in accordance with Claim 1 wherein the cementatory network is substantially constituted by lactose and said discrete particles comprise estazolam enveloped in hydrogenated cottonseed oil.

8. The method in accordance with Claim 1 wherein the carbohydrate is lactose, the active ingredient is estazolam, the triglyceride is hydrogenated cottonseed oil, the polymer is polyethylene glycol of molecular weight 300, and the emulsifier is lecithin, wherein the carbohydrate is 94 percent of the total weight of the tablet.

9. A method of manufacturing a solid pharmaceutical dosage in tablet triturate form, suitable for oral administration which comprises:
admixing a particulate water-soluble but ethanol insoluble carbohydrate with discrete particles containing a solid, water-soluble but triglyceride-insoluble active ingredient, a polymer, an emulsifier, and sodium bicarbonate enveloped in a triglyceride coating, said triglyceride coating being insoluble in water, ethanol, saliva or combinations thereof and constituting a major portion of the weight of each said discrete particle; and,
tabletting the produced admixture.

10. A method of manufacturing a tablet triturate dosage form, comprising:
admixing a particulate water-soluble but ethanol-insoluble carbohydrate with discrete particles containing a solid, water-soluble but triglyceride-insoluble active ingredient enveloped in a triglyceride coating.

## Patentansprüche (Patentansprüche für folgende(n) Vertragsstaat(en): BE, CH, DE, FR, GB, IT, LI, NL, SE)

1. Feste, pharmazeutische, zur oralen Verabreichung geeignete Dosierung in pulverisierter Form als Tablette, umfassend:
ein poröses, kittendes Netzwerk, das im wesentlichen aus einem wasserlöslichen, aber Ethanol-unlöslichen Kohlehydrat besteht; und
diskrete Teilchen, umfassend einen festen, wasserlöslichen, aber Triglycerid-unlöslichen, aktiven Bestandteil, die einen Triglycerid-Überzug aufweisen, die im wesentlichen in dem genannten Netzwerk einheitlich verteilt sind, wobei der genannte Triglycerid-Überzug in Wasser, Ethanol, Speichel oder Mischungen davon unlöslich ist;
wobei der genannte Triglycerid-Überzug einen wesentlichen Teil des Gewichts eines jeden genannten diskreten Teilchens ausmacht.

2. Dosierungsform nach Anspruch 1, bei der der aktive Bestandteil Estazolam ist.

3. Dosierungsform nach Anspruch 2, bei der die diskreten Teilchen weiterhin einen Emulgator und ein Polymeres enthalten.

4. Dosierungsform nach Anspruch 3, bei der die diskreten Teilchen weiterhin Natriumbicarbonat enthalten.

5. Dosierungsform nach Anspruch 4, bei der die diskreten Teilchen Estazolam als aktiven Bestandteil, hydriertes Baumwollsamenöl als Triglycerid, Polyethylenglycol mit einem Molekulargewicht von 300 als Polymeres, Lecithin als Emulgator und Natriumbicarbonat enthalten.

6. Feste, pharmazeutische, zur oralen Verabreichung geeignete Dosierung in pulverisierter Form als Tablette, umfassend:
ein poröses, kittendes Netzwerk, das im wesentlichen aus einem wasserlöslichen, aber Ethanol-unlöslichen Kohlehydrat besteht; und
diskrete Teilchen, umfassend einen festen, wasserlöslichen, aber Triglycerid-unlöslichen, aktiven Bestandteil, ein Polymeres, Natriumbicarbonat und einen Emulgator, wobei die Teilchen einen Triglycerid-Überzug aufweisen, wobei der genannte Triglycerid-Überzug in Wasser, Ethanol, Speichel oder Mischungen davon unlöslich ist; und wobei
der genannte Triglycerid-Überzug einen größeren Anteil des Gewichts eines jeden beschichteten Teilchens ausmacht, wobei die Triglycerid-beschichteten Teilchen im wesentlichen einheitlich über das genannte Netzwerk verteilt sind.

7. Dosierungsform nach Anspruch 1, bei der das kittende Netzwerk im wesentlichen aus Lactose besteht und die genannten diskreten Teilchen mit hydriertem Baumwollsamenöl beschichtetes Estazolam enthalten.

8. Dosierungsform nach Anspruch 1, bei der das Kohlehydrat Lactose, der aktive Bestandteil Estazolam, das Triglycerid hydriertes Baumwollsamenöl, das Polymere Polyethylenglycol mit einem Molekulargewicht von 300 und der Emulgator Lecithin sind, worin das Kohlehydrat 94 % des Gesamtgewichts der Tablette ausmacht.

9. Dosierungsform nach Anspruch 1, bei der das Gewichtsverhältnis Kohlehydrat:diskrete Teilchen 4:1 bis 100:1 beträgt.

10. Dosierungsform nach Anspruch 1, bei der der Triglycerid-Überzug den Geschmack des aktiven Bestandteiles überdeckt.

11. Verfahren zur Herstellung einer festen, pharmazeutischen, zur oralen Verabreichung geeigneten Dosierung in pulverisierter Form als Tablette, umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen, aktiven Bestandteil enthalten, der mit einem Triglycerid-Überzug beschichtet ist, wobei der genannte Triglycerid-Überzug in Wasser, Ethanol, Speichel oder Gemischen davon unlöslich ist und einen größeren Teil des Gewichts eines jeden genannten diskreten Teilchens ausmacht;
Vermischen des erhaltenen Gemisches mit einem leicht flüchtigen, flüssigen Bindemittel, in dem das genannte Kohlehydrat eine begrenzte Löslichkeit in einem Ausmaß aufweist, das zur Bildung einer formbaren Masse ausreicht;
Tablettieren der genannten, formbaren Masse; und
Trocknen der genannten geformten Tabletten zu einer festen Struktur.

12. Verfahren zur Herstellung einer festen, pharmazeutischen, zur oralen Verabreichung geeigneten Dosierung in pulverisierter Form als Tablette umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen, aktiven Bestandteil, ein Polymeres, einen Emulgator und Natriumbicarbonat, beschichtet mit einem Triglycerid-Überzug, enthalten, wobei der genannte Triglycerid-Überzug in Wasser, Ethanol, Speichel oder Mischungen davon unlöslich ist und einen größeren Teil des Gewichtes eines jeden genannten, diskreten Teilchens ausmacht; und
Tablettieren des hergestellten Gemisches.

13. Verfahren zur Herstellung einer pulverisierter Form als Tablette, umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen, aktiven Bestandteil, beschichtet mit einem Triglycerid-Überzug, enthalten.

## Patentansprüche (Patentansprüche für folgende(n) Vertragsstaat(en): ES, GR)

1. Verfahren zur Herstellung einer festen, pharmazeutischen, zur oralen Verabreichung geeigneten Dosierung in pulverisierter Form als Tablette, umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen, aktiven Bestandteil umfassen, der mit einem Triglycerid-Überzug beschichtet ist, wobei der genannte Triglycerid-Überzug in Wasser, Ethanol, Speichel oder Mischungen davon unlöslich ist und einen größeren Teil des Gewichtes eines jeden genannten diskreten Teilchens ausmacht;
Kombinieren der resultierenden Mischung mit einem flüssigen Bindemittel, das verflüchtigt werden kann, in dem das genannte Kohlehydrat eine begrenzte Löslichkeit aufweist, in einer Menge, die zur Bildung einer formbaren Masse ausreicht;
Formen der genannten formbaren Masse zu Tabletten; und Trocknen der genannten, geformten Tabletten zu einer festen Struktur.

2. Verfahren nach Anspruch 1, bei dem der aktive Bestandteil Estazolam ist.

3. Verfahren nach Anspruch 2, bei dem die diskreten Teilchen ferner einen Emulgator und ein Polymeres enthalten.

4. Verfahren nach Anspruch 3, bei dem die diskreten Teilchen außerdem Natriumbicarbonat enthalten.

5. Verfahren nach Anspruch 4, bei dem die diskreten Teilchen Estazolam als aktiven Bestandteil, hydriertes Baumwollsamenöl als Triglycerid, Polyethylenglycol mit einem Molekulargewicht von 300 als Polymeres, Lecithin als Emulgator und Natriumbicarbonat enthalten.

6. Verfahren nach Anspruch 1, bei dem das Gewichtsverhältnis Kohlehydrat:diskrete Teilchen 4:1 bis 100:1 beträgt.

7. Verfahren nach Anspruch 1, bei dem das kittende Netzwerk im wesentlichen aus Lactose besteht und die genannten diskreten Teilchen mit hydriertem Baumwollsamenöl beschichtetes Estazolam enthalten.

8. Verfahren nach Anspruch 1, bei dem das Kohlehydrat Lactose, der aktive Bestandteil Estazolam, das Trigylcerid hydriertes Baumwollsamenöl, das Polymere Polyethylenglycol mit einem Molekulargewicht von 300 und der Emulgator Lecithin sind, bei dem das Kohlehydrat 94% des Gesamtgewichts der Tablette ausmacht.

9. Verfahren zur Herstellung einer festen, pharmazeutischen, zur oralen Verabreichnung geeigneten Dosierung in pulverisierter Form als Tablette, umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen, aktiven Bestandteil, ein Polymeres, einen Emulgator und von einem Triglycerid-Überzug umhülltes Natriumbicarbonat enthalten, wobei der genannte Triglycerid-Überzug in Wasser, Ethanol, Speichel oder Mischungen davon unlöslich ist und einen größeren Teil des Gewichtes eines jeden genannten diskreten Teilchens darstellt; und
Tablettieren der hergestellten Mischung.

10. Verfahren zur Herstellung einer Dosierung in pulverförmiger Form als Tablette, umfassend:
Vermischen eines teilchenförmigen, wasserlöslichen, aber Ethanol-unlöslichen Kohlehydrates mit diskreten Teilchen, die einen festen, wasserlöslichen, aber Triglycerid-unlöslichen von einem Triglycerid-Überzug beschichteten, aktiven Bestandteil enthalten.

## Revendications (Revendications pour l'(les) Etat(s) contractant(s) suivant(s): BE, CH, DE, FR, GB, IT, LI, NL, SE)

1. Présentation pharmaceutique solide sous forme de trituration en comprimé, convenant à l'administration orale et comprenant
un réseau cémentatoire poreux essentiellement constitué par un glucide soluble dans l'eau mais insoluble dans l'éthanol et
des particules discrètes comprenant un principe actif solide, soluble dans l'eau mais insoluble dans les triglycérides, ayant un enrobage triglycéridique, distribuées de façon sensiblement uniforme à l'intérieur dudit réseau, ledit enrobage triglycéridique étant insoluble dans l'eau, l'éthanol, la salive ou leurs combinaisons ;
ledit enrobage triglycéridique constituant une majeure partie du poids de chacune desdites particules discrètes.

2. Présentation selon la revendication 1, dans laquelle le principe actif est de l'estazolam.

3. Présentation selon la revendication 2, dans laquelle les particules discrètes comprennent en outre un émulsifiant et un polymère.

4. Présentation selon la revendication 3, dans laquelle les particules discrètes comprennent en outre du bicarbonate de sodium.

5. Présentation selon la revendication 4, dans laquelle les particules discrètes comprennent de l'estazolam comme principe actif, de l'huile de coton hydrogénée comme triglycéride, du polyéthylèneglycol de poids moléculaire égal à 300 comme polymère, de la lécithine comme émulsifiant et du bicarbonate de sodium.

6. Présentation pharmaceutique solide sous forme de trituration en comprimé, convenant à l'administration orale et comprenant
un réseau cémentatoire poreux constitué essentiellement par un glucide soluble dans l'eau mais insoluble dans l'éthanol et
des particules discrètes comprenant un principe actif solide, soluble dans l'eau mais insoluble dans les triglycérides, un polymère, du bicarbonate de sodium et un émulsifiant, lesdites particules ayant un enrobage triglycéridique, ledit enrobage triglycéridique étant insoluble dans l'eau, l'éthanol, la salive ou leurs combinaisons et
ledit enrobage triglycéridique constituant une majeure partie du poids de chacune des particules enrobées, lesdites particules enrobées par le triglycéride étant distribuées de façon sensiblement uniforme à l'intérieur dudit réseau.

7. Présentation selon la revendication 1, dans laquelle le réseau cémentatoire est essentiellement constitué par du lactose et lesdites particules discrètes comprennent de l'estazolam enrobé d'huile de coton hydrogénée.

8. Présentation selon la revendication 1, dans laquelle le glucide est du lactose, le principe actif est de l'estazolam, le triglycéride est de l'huile de coton hydrogénée, le polymère est du polyéthylèneglycol de poids moléculaire égal à 300 et l'émulsifiant est de la lécithine, dans laquelle le glucide représente 94 % du poids total du comprimé.

9. Présentation selon la revendication 1, dans laquelle le rapport pondéral glucide:particules discrètes est compris entre 4:1 et 100:1.

10. Présentation selon la revendication 1, dans laquelle l'enrobage triglycéridique masque la saveur du principe actif.

11. Procédé de production d'une présentation pharmaceutique solide sous forme de trituration en comprimé, convenant à l'administration orale, lequel comprend :
le mélange d'un glucide particulaire, soluble dans l'eau mais insoluble dans l'éthanol, avec des particules discrètes contenant un principe actif solide, soluble dans l'eau mais insoluble dans les triglycérides, doté d'un enrobage triglycéridique, ledit enrobage triglycéridique étant insoluble dans l'eau, l'éthanol, la salive ou leurs combinaisons et constituant une majeure partie du poids de chacune desdites particules discrètes ;
la combinaison du mélange obtenu avec un liant liquide volatilisable dans lequel ledit glucide possède une solubilité limitée, en une quantité suffisante pour former une masse moulable ;
le façonnage de ladite masse moulable en comprimés et le séchage desdits comprimés façonnés en une structure rigide.

12. Procédé de production d'une présentation pharmaceutique solide sous forme de trituration en comprimé, convenant à l'administration orale, lequel comprend :
le mélange d'un glucide particulaire, soluble dans l'eau mais insoluble dans l'éthanol, avec des particules discrètes contenant un principe actif solide, soluble dans l'eau mais insoluble dans les triglycérides, un polymère, un émulsifiant et du bicarbonate de sodium dotés d'un enrobage triglycéridique, ledit enrobage triglycéridique étant insoluble dans l'eau, l'éthanol, la salive ou leurs combinaisons et représentant une majeure partie du poids de chacune desdites particules discrètes et
la fabrication de comprimés à l'aide du mélange produit.

13. Procédé de production d'une présentation d'une trituration en comprimé, comprenant :
le mélange d'un glucide particulaire, soluble dans l'eau mais insoluble dans l'éthanol, avec des particules discrètes contenant un principe actif solide, soluble dans l'eau mais insoluble dans les triglycérides, doté d'un enrobage triglycéridique.

## Revendications (Revendications pour l'(les) Etat(s) contractant(s) suivant(s): ES, GR)

1. Procédé de production d'une présentation pharmaceutique solide sous forme de trituration en comprimé, convenant à l'administration orale, lequel comprend :
le mélange d'un glucide particulaire, soluble dans l'eau mais insoluble dans l'éthanol, avec des particules discrètes comprenant un principe actif solide, soluble dans l'eau mais insoluble dans les triglycérides, doté d'un enrobage triglycéridique, ledit enrobage triglycéridique étant insoluble dans l'eau, l'éthanol, la salive ou leurs combinaisons et constituant une majeure partie du poids de chacune desdites particules discrètes ;
la combinaison du mélange obtenu avec un liant liquide volatilisable dans lequel ledit glucide a une solubilité limitée, en une quantité suffisante pour former une masse moulable ;
le façonnage de ladite masse moulable en comprimés et le séchage desdits comprimés façonnés en une structure rigide.

2. Procédé selon la revendication 1, dans lequel le principe actif est de l'estazolam.

3. Procédé selon la revendication 2, dans lequel les particules discrètes comprennent en outre un émulsifiant et un polymère.

4. Procédé selon la revendication 3, dans lequel les particules discrètes comprennent en outre du bicarbonate de sodium.

5. Procédé selon la revendication 4, dans lequel les particules discrètes comprennent de l'estazolam comme principe actif, de l'huile de coton hydrogénée comme triglycéride, du polyéthylèneglycol de poids moléculaire égal à 300 comme polymère, de la lécithine comme émulsifiant et du bicarbonate de sodium.

6. Procédé selon la revendication 1, dans lequel le rapport pondéral glucide:particules discrètes est compris entre 4:1 et 100:1.

7. Procédé selon la revendication 1, dans lequel le réseau cémentatoire est essentiellement constitué par du lactose et lesdites particules discrètes comprennent de l'estazolam enrobé d'huile de coton hydrogénée.

8. Procédé selon la revendication 1, dans lequel le glucide est du lactose, le principe actif est de l'estazolam, le triglycéride est de l'huile de coton hydrogénée, le polymère est du polyéthylèneglycol de poids moléculaire égal à 300 et l'émulsifiant est de la lécithine, dans lequel le glucide représente 94 % du poids total du comprimé.

9. Procédé de production d'une présentation pharmaceutique soluble sous forme de trituration en comprimé, convenant à l'administration orale, lequel comprend :
le mélange d'un glucide particulaire, soluble dans l'eau mais insoluble dans l'éthanol, avec des particules discrètes contenant un principe actif solide, soluble dans l'eau mais insoluble dans les triglycérides, un polymère, un émulsifiant et du bicarbonate de sodium dotés d'un enrobage triglycéridique, ledit enrobage triglycéridique étant insoluble dans l'eau, l'éthanol, la salive ou leurs combinaisons et constituant une majeure partie du poids de chacune desdites particules discrètes ; et
la fabrication de comprimés à l'aide du mélange produit.

10. Procédé de production d'une présentation d'une substance triturée en comprimé, comprenant :
le mélange d'un glucide particulaire, soluble dans l'eau mais insoluble dans l'éthanol, avec des particules discrètes contenant un principe actif solide, soluble dans l'eau mais insoluble dans les triglycérides, doté d'un enrobage triglycéridique.
